(19) Europäisches Patentamt
European Patent Office
Office européen des brevets

(11) Veröffentlichungsnummer : **0 029 117**
**B1**

(12) # EUROPÄISCHE PATENTSCHRIFT

(45) Veröffentlichungstag der Patentschrift : **20.04.83**

(21) Anmeldenummer : **80106152.4**

(22) Anmeldetag : **10.10.80**

(51) Int. Cl.³ : **C 07 D471/22**, A 61 K 31/475 // (C07D471/22, 221/00, 221/00, 221/00, 209/00)

(54) **10-Bromsandwicin und 10-Bromisosandwicin sowie deren Säureadditionssalze mit pharmakologisch annehmbaren Säuren, Verfahren zu deren Herstellung und deren Verwendung.**

(30) Priorität : **13.10.79 DE 2941531**

(43) Veröffentlichungstag der Anmeldung : **27.05.81 Patentblatt 81/21**

(45) Bekanntmachung des Hinweises auf die Patenterteilung : **20.04.83 Patentblatt 83/16**

(84) Benannte Vertragsstaaten : **AT BE CH DE FR GB IT LI LU NL SE**

(56) Entgegenhaltungen :
**DE A 2 611 162**
**DE A 2 701 417**

**JOURNAL OF THE CHEMICAL SOCIETY, 1954, Part I, London F.A.L. ANET et al. « Ajmaline. Part I » Seiten 1242 bis 1260**

**Chemical Abstracts, Band 79, Nr. 9, 3. September 1973 Columbus, Ohio, USA, V. AHMAD et al. « Ajmaline series » Seite 394, Spalte 1, Abstract Nr 53652b**

**Die Akte enthält technische Angaben, die nach dem Eingang der Anmeldung eingereicht wurden und die nicht in dieser Patentschrift enthalten sind.**

(73) Patentinhaber : **Kali-Chemie Pharma GmbH**
**Hans-Böckler-Allee 20 Postfach 220**
**D-3000 Hannover 1 (DE)**

(72) Erfinder : **Kehrbach, Wolfgang Dipl.-Chem., Dr.rer.nat.**
**Altenbekener Damm 41**
**D-3000 Hannover 1 (DE)**
Erfinder : **Wegener, Joachim Dipl.-Chem., Dr.rer.nat.**
**Mühlenstrasse 41**
**D-3201 Algermissen (DE)**

(74) Vertreter : **Lauer, Dieter, Dr.**
**c/o Kali-Chemie Aktiengesellschaft Postfach 220**
**D-3000 Hannover 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 029 117 B1

10-Bromsandwicin und 10-Bromisosandwicin, sowie deren Säureadditionssalze mit pharmakologisch annehmbaren Säuren, Verfahren zu deren Herstellung und deren Verwendung

Die Erfindung betrifft 10-Bromsandwicin und 10-Bromisosandwicin, sowie deren Säureadditionssalze mit pharmakologisch annehmbaren Säuren, Verfahren zu deren Herstellung und deren Verwendung.

Es ist bekannt, daß sich $N_b$-quartäre Derivate von Sandwicin und Isosandwicin durch vorteilhafte pharmakologische Wirkungen, insbesondere herzrhythmisierende Wirkungen auszeichnen (DE-OS 26 11 162). Es wurde gefunden, daß sich die vorteilhaften pharmakologischen Eigenschaften der $N_b$-quartären Derivate von Sandwicin und Isosandwicin weiter verbessern lassen durch Einführung eines Brom-Atoms in 10-Stellung des Sandwicin- bzw. Isosandwicin-Gerüstes (siehe EP-A-80106151) und daß 10-Bromsandwicin und 10-Bromisosandwicin selbst schon vorteilhafte pharmakologische Wirkungen zeigen.

Gegenstand der Erfindung sind demnach 10-Bromsandwicin der Formel I

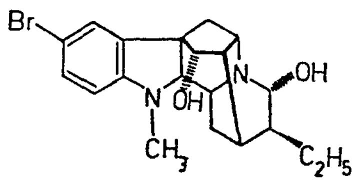

(I)

und 10-Bromisosandwicin der Formel II

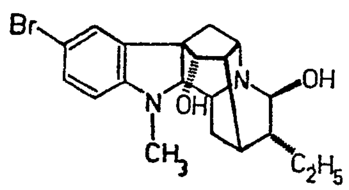

(II)

sowie deren Säureadditionssalze mit pharmakologisch annehmbaren Säuren.

Gegenstand der Erfindung ist auch die Verwendung von 10-Bromsandwicin und 10-Bromisosandwicin als Zwischenprodukte zur Herstellung von Arzneistoffen, insbesondere von $N_b$-quartären 10-Bromsandwicin- und 10-Bromisosandwicin-Derivaten (vergl. parallele Anmeldung EP-A-80106151) sowie die Verwendung von 10-Bromsandwicin und 10-Bromisosandwicin sowie deren Säureadditionssalze mit pharmakologisch annehmbaren Säuren als Wirkstoff in Arzneimitteln.

Gegenstand der Erfindung ist schließlich auch ein Verfahren zur Herstellung von 10-Bromsandwicin und 10-Bromisosandwicin.

Die Herstellung von 10-Bromsandwicin erfolgt durch Bromierung von Sandwicin (M. Gorman, N. Neuss, C. Djerassi, J.P. Kutney und P.J. Scheurer, Tetrahedron, Vol. 1 (1957), 328-337) mit einem geeigneten Bromierungsmittel, zweckmäßigerweise in einem inerten Lösungsmittel.

Als Bromierungsmittel haben sich elementares Brom, Kupfer(II)-bromid und insbesondere 2,4,4,6-Tetrabrom-2,5-cyclohexadien-1-on, als besonders geeignet erwiesen.

Im Fall der Bromierung mit elementarem Brom haben sich ein Gemisch aus Methylenchlorid und Methanol als Lösungsmittel und das Arbeiten bei etwa 0 °C als besonders vorteilhaft erwiesen.

Wenn man mit Kupfer(II)-bromid bromiert, ist es vorteilhaft in Dimethylformamid bei Raumtemperatur zu arbeiten.

Die Bromierung mit 2,4,4,6-Tetrabrom-2,5-cyclohexadien-1-on in einem Gemisch aus Methylenchlorid und Tetrahydrofuran und unter Kühlung, vorzugsweise bei − 5 bis − 10 °C, zeigt die besten Ergebnisse.

10-Bromsandwicin kann durch Isomerisierung in 10-Bromisosandwicin übergeführt werden, wobei die Isomerisierung unter alkalischen Bedingungen, bevorzugt in alkoholischer Alkalilauge, insbesondere methanolischer Kalilauge vorgenommen wird. Zweckmäßigerweise isomerisiert man bei erhöhter Temperatur, vorteilhafterweise bei der Siedetemperatur des Lösungsmittels.

Die Erfindung wird durch die nachfolgenden Beispiele näher erläutert.

Beispiele 1-8

10-Bromsandwicin und dessen Salze

Beispiel 1

10-Bromsandwicin-hydrobromid

3 g Sandwicin wurden in 50 ml eines Gemisches aus Methylenchlorid und Methanol gelöst und unter Rühren und Eiskühlung sehr langsam mit einer Lösung von 1,45 g Brom in 100 ml Methanol versetzt. Nach dem Einengen wurde aus Aceton kristallisiert.
Ausbeute : 3,4 g (76 %)
Fp. : 210 °C
reine n-Form

Beispiel 2

10-Bromsandwicin

12,4 g 10-Bromsandwicin-hydrobromid wurden unter Erwärmen in 100 ml Methanol gelöst und nach dem Abkühlen tropfenweise bis zur vollständigen Fällung mit verdünnter Sodalösung versetzt. 10-Bromsandwicin wurde abgesaugt und getrocknet.
Ausbeute : 9 g (87 %)
Fp. : 204 °C
reine n-Form

Beispiel 3

10-Bromsandwicin

48,2 g 10-Bromsandwicinhydrobromid wurden mit verdünnter Sodalösung versetzt und dreimal mit Methylenchlorid, dem etwas Methanol zugesetzt war, extrahiert. Die organische Phase wurde mit Wasser gewaschen, getrocknet und zur Trockene eingeengt.
Ausbeute : 39,6 g (99 %)
Fp. : 204 °C
reine n-Form

Beispiel 4

10-Bromsandwicin

12,5 g Sandwicin und 19 g Kupfer (II)-bromid wurden in Dimethylformamid gelöst und 4 Stunden bei Raumtemperatur gerührt. Nach dem Abdestillieren des Lösungsmittels wurde der Rückstand mit Wasser aufgenommen, mit Ammoniaklösung versetzt und mehrfach mit Essigester extrahiert. Die organische Phase wurde getrocknet, eingeengt, der Rückstand in Methanol aufgenommen und das 10-Bromsandwicin durch Zugabe von verdünnter Sodalösung gefällt.
Ausbeute : 4,5 g (29 %)
Fp. : 204 °C
reine n-Form

Beispiel 5

10-Bromsandwicin-hydrochlorid

1 g Sandwicin und 1,5 g Kupfer(II)-bromid wurden in Dimethylformamid gelöst und bei Raumtemperatur gerührt. Nach 4 Stunden wurde das Lösungsmittel im Vakuum abdestilliert, der Rückstand in Wasser aufgenommen, mit Ammoniak versetzt und mehrfach mit Essigester extrahiert. Die organische Phase wurde getrocknet, eingeengt, der Rückstand in Methanol aufgenommen, mit methanolischer Salzsäure versetzt und in Äther eingetropft. Das ausgefallene Hydrochlorid wurde abfiltriert, mit Äther gewaschen und getrocknet.
Ausbeute : 0,9 g (66 %)
Fp. : 268-70 °C
reine n-Form

Beispiel 6

10-Bromsandwicin

13,2 g Sandwicin wurden in 400 ml Tetrahydrofuran, 100 ml Methylenchlorid und wenig Methanol

gelöst und bei – 10 °C in kleinen Portionen 16,6 g 2,4,4,6-Tetrabrom-2,5-cyclohexadien-1-on zugefügt. Nach 30-minütigem Rühren bei – 10 °C ließ man langsam auf Raumtemperatur erwärmen und extrahierte zur Entfernung des Phenols zweimal mit 2n Natronlauge. Nach zweimaligem Waschen mit Wasser wurde eingeengt, in Methanol aufgenommen und das entstandene 10-Bromsandwicin durch Zugabe von Wasser ausgefällt.

Ausbeute : 15,1 g (92 %)
Fp. : 204 °C
reine n-Form

Durch Ansäuern der wäßrigen Phase und Extraktion mit Methylenchlorid kann 2,4,6-Tribromphenol zurückgewonnen werden, das als Ausgangsmaterial für die Synthese von 2,4,4,6-Tetrabrom-2,5-cyclohexadien-1-on wieder zur Verfügung steht.

Beispiel 7

10-Bromsandwicin

26,6 g Sandwicin wurden in 2 Litern eines Gemischs aus Tetrahydrofuran und Methylenchlorid (4 : 1 ; V : V) gelöst und bei – 5 °C unter Rühren portionsweise mit 33 g 2,4,4,6-Tetrabrom-2,5-cyclohexadien-1-on versetzt. Nach der letzten Zugabe wurde noch 30 Minuten bei – 5 °C bis – 10 °C gerührt, auf Raumtemperatur erwärmt, mit weiterem Methylenchlorid versetzt, zweimal mit 2n-Natronlauge und anschließend zweimal mit Wasser gewaschen, die organische Phase eingeengt, in 500 ml Methanol aufgenommen und zur methanolischen Lösung langsam Wasser zugetropft, bis die Fällung des 10-Bromsandwicins vollständig war. Das Produkt wurde abgesaugt, mit Wasser, dann mit kaltem Aceton gewaschen und getrocknet.

Ausbeute : 30,1 g (91 %)
Fp. : 204 °C
reine n-Form

Durch Ansäuern der wäßrig alkalischen Extrakte konnte 2,4,6-Tribromphenol gewonnen werden, das nach Umkristallisation aus Petroläther erneut zur Darstellung von 2,4,4,6-Tetrabrom-2,5-cyclohexadien-1-on verwendet wurde.

Beispiel 8

10-Bromsandwicinium-hydrogentartrat

2 g 10-Bromsandwicin und 0,74 g L(+)-Weinsäure wurden in 20 ml Methanol und 5 ml Methylenchlorid gelöst, auf die Hälfte eingeengt und das Hydrogentartrat durch Eintropfen in 200 ml Äther gefällt.

Ausbeute : 2,4 g (88 %)
Fp. : 208 °C
reine n-Form

Beispiele 9-11

10-Bromisosandwicin und dessen Salze

Beispiel 9

10-Bromisosandwicin

15 g 10-Bromsandwicin und 20 g Kaliumhydroxid wurden in 700 ml Methanol gelöst und 8 Stunden unter Rückfluß erhitzt. Nach dem Verdünnen mit 400 ml Wasser wurde dreimal mit Methylenchlorid extrahiert, getrocknet, eingeengt und aus Methanol kristallisiert.

Ausbeute : 10,5 g (70 %)
Fp. : 173-5 °C
reine iso-Form

Das in der Mutterlauge verbleibende Gemisch von 10-Bromsandwicin und wenig 10-Bromisosandwicin kann erneut isomerisiert werden.

Beispiel 10

10-Bromisosandwicinium-hydrogentartrat

2,5 g 10-Bromisosandwicin wurden in 200 ml Essigester gelöst und tropfenweise mit einer Lösung von 0,92 g L(+)- Weinsäure in 7 ml Aceton versetzt. Das ausgefallene Hydrogentartrat wurde abfiltriert und mit Essigester gewaschen.

Ausbeute : 3,1 g (90 %)
Fp. : 210 °C
reine iso-Form

## Beispiel 11

10-Bromisosandwicinium-dihydrogencitrat

1 g 10-Bromisosandwicin wurde in 15 ml Essigsäureäthylester gelöst und tropfenweise mit einer Lösung von 0,57 g Citronensäure-1-hydrat in 3 ml Aceton und 4 ml Essigsäureäthylester versetzt. Das ausgefallene Dihydrogencitrat wurde abgesaugt, mit Essigsäureäthylester gewaschen und getrocknet.
Ausbeute : 1,1 g (75 %)
Fp. : 220-2 °C
reine iso-Form

## Beispiele 12-13

Alkylierung von 10-Bromsandwicin

12a) $N_b$-Methyl-10-bromsandwiciniumjodid

12 g 10-Bromsandwicin und 13 ml Methyljodid wurden in 500 ml Acetonitril 8 Stunden unter Rückfluß erhitzt. Der ausgefallene Niederschlag wurde abfiltriert und mit Essigester gewaschen.
Ausbeute : 9,5 g (59 %)
Fp. : 240-2 °C
reine n-Form

12b) $N_b$-Methyl-10-bromsandwicinium-hydrogentartrat

8,7 g $N_b$-Methyl-10-bromsandwiciniumjodid wurden mit verdünnter Sodalösung aufgenommen und mit Essigester extrahiert. Zu dieser Lösung tropfte man eine Lösung von 2,4 g L(+)-Weinsäure in Aceton, filtrierte den ausgefallenen Niederschlag ab und wusch mit Essigester.
Ausbeute : 8,0 g (52 %)
Fp. : 166-70 °C
reine n-Form

13a) $N_b$-n-Propyl-10-bromsandwiciniumjodid

Die Alkylierung mit n-Propyljodid erfolgte analog Beispiel 12a.
Ausbeute : 67 %
Fp. : 270 °C (dec)
reine n-Form

13b) $N_b$-n-Propyl-10-bromsandwicinium-hydrogentartrat

Die Umwandlung ins Hydrogentartrat erfolgte analog Beispiel 12b.
Ausbeute : 57 %
Fp. : 153-5 °C
reine n-Form

## Beispiel 14

Alkylierung von 10-Bromisosandwicin

14a) $N_b$-n-Propyl-10-bromisosandwiciniumjodid

Die Darstellung erfolgte analog Beispiel 12a.
Ausbeute : 87 %
Fp. : 265 °C (dec)
reine iso-Form

14b) $N_b$-n-Propyl-10-bromisosandwiciniumhydrogentartrat

12 g $N_b$-n-Propyl-10-bromisosandwiciniumjodid wurden mit 300 ml verdünnter Sodalösung aufgenommen, dreimal mit Methylenchlorid extrahiert, die organische Phase getrocknet, stark eingeengt und

mit 600 ml Essigester aufgenommen. Zu dieser Lösung tropfte man eine konzentrierte Lösung von 3,1 g L(+)-Weinsäure in Aceton. Das ausgefällte Hydrogentartrat wurde abfiltriert und mit Essigester gewaschen.

Ausbeute : 81 %
Fp. : 149-51 °C
reine iso-Form

**Ansprüche**

1. 10-Bromsandwicin der Formel I

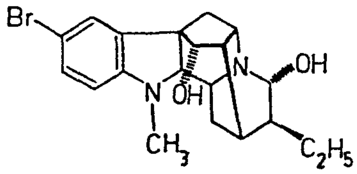

(I)

und dessen Säureadditionssalze mit pharmakologisch annehmbaren Säuren.

2. 10-Bromisosandwicin der Formel II

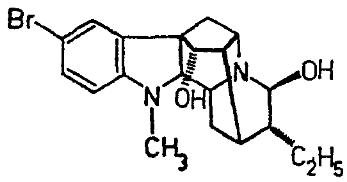

(II)

und dessen Säureadditionssalze mit pharmakologisch annehmbaren Säuren.

3. Verfahren zur Herstellung von 10-Bromsandwicin, dadurch gekennzeichnet, daß man Sandwicin mit einem geeigneten Bromierungsmittel bromiert.

4. Verfahren nach Anspruch 3, dadurch gekennzeichnet, daß man in einem inerten Lösungsmittel bromiert.

5. Verfahren nach Anspruch 3 oder 4, dadurch gekennzeichnet, daß man mit elementarem Brom bromiert.

6. Verfahren nach Anspruch 3 oder 4, dadurch gekennzeichnet, daß man mit wasserfreiem Kupfer(II)-bromid bromiert.

7. Verfahren nach Anspruch 3 oder 4, dadurch gekennzeichnet, daß man mit 2,4,4,6-Tetrabrom-2,5-cyclohexadien-1-on bromiert.

8. Verfahren nach einem der Ansprüche 3, 4 oder 5, dadurch gekennzeichnet, daß man in einem Gemisch aus Methylenchlorid und Methanol bromiert.

9. Verfahren nach einem der Ansprüche 3, 4 oder 6, dadurch gekennzeichnet, daß man in Dimethylformamid bromiert.

10. Verfahren nach einem der Ansprüche 3, 4 oder 7, dadurch gekennzeichnet, daß man in einem Gemisch aus Tetrahydrofuran und Methylenchlorid, dem Methanol zugesetzt sein kann, bromiert.

11. Verfahren nach einem der Ansprüche 5 oder 8, dadurch gekennzeichnet, daß man bei 0 °C bromiert.

12. Verfahren nach einem der Ansprüche 6 oder 9, dadurch gekennzeichnet, daß man bei Raumtemperatur bromiert.

13. Verfahren nach einem der Ansprüche 7 oder 10, dadurch gekennzeichnet, daß man unter Kühlung, vorzugsweise bei − 5 bis − 10 °C bromiert.

14. Verfahren zur Herstellung von 10-Bromisosandwicin, dadurch gekennzeichnet, daß man 10-Bromsandwicin unter alkalischen Bedingungen isomerisiert.

15. Verfahren nach Anspruch 14, dadurch gekennzeichnet, daß man in alkoholischer Alkalilauge isomerisiert.

16. Verfahren nach Anspruch 15, dadurch gekennzeichnet, daß man mit methanolischer Kalilauge isomerisiert.

17. Verfahren nach Anspruch 16, dadurch gekennzeichnet, daß man bei der Siedetemperatur des Lösungsmittels isomerisiert.

18. Verwendung von 10-Bromsandwicin oder 10-Bromisosandwicin zur Herstellung von $N_b$-quartären 10-Bromsandwicin- oder 10-Bromisosandwicin-Derivaten.

19. 10-Bromsandwicin oder 10-Bromisosandwicin und deren Säureadditionssalze mit pharmakologisch annehmbaren Säuren zur Verwendung als Arzneimittel.

**Claims**

1. 10-Bromosandwicin of the formula I

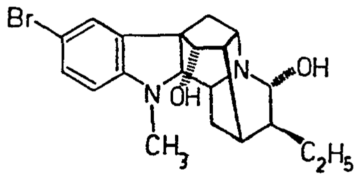

(I)

and acid addition salts thereof with pharmacologically acceptable acids.

2. 10-Bromoisosandwicin of the formula II

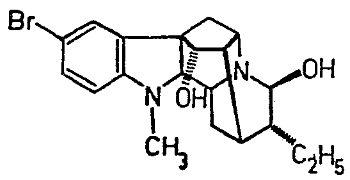

(II)

and acid addition salts thereof with pharmacologically acceptable acids.

3. Process for the preparation of 10-bromosandwicin, characterised in that sandwicin is brominated with a suitable brominating agent.

4. Process according to Claim 3, characterised in that the bromination is carried out in an inert solvent.

5. Process according to Claim 3 or 4, characterised in that the bromination is carried out with elemental bromine.

6. Process according to Claim 3 or 4, characterised in that the bromination is carried out with anhydrous copper-II bromide.

7. Process according to Claim 3 or 4, characterised in that the bromination is carried out with 2,4,4,6-tetrabromo-2,5-cyclohexadien-1-one.

8. Process according to one of Claims 3, 4 or 5, characterised in that the bromination is carried out in a mixture of methylene chloride and methanol.

9. Process according to one of Claims 3, 4 or 6, characterised in that the bromination is carried out in dimethylformamide.

10. Process according to one of Claims 3, 4 or 7, characterised in that the bromination is carried out in a mixture of tetrahydrofuran and methylene chloride, to which methanol may be added.

11. Process according to one of Claims 5 or 8, characterised in that the bromination is carried out at 0 °C.

12. Process according to one of Claims 6 or 9, characterised in that the bromination is carried out at room temperature.

13. Process according to one of Claims 7 or 10, characterised in that the bromination is carried out with cooling, preferably at − 5 to − 10 °C.

14. Process for the preparation of 10-bromoisosandwicin, characterised in that the 10-bromosandwicin is isomerised under alkaline conditions.

15. Process according to Claim 14, characterised in that the isomerisation is carried out in alcoholic alkali metal hydroxide solution.

16. Process according to Claim 15, characterised in that the isomerisation is carried out with methanolic potassium hydroxide solution.

17. Process according to Claim 16, characterised in that the isomerisation is carried out at the boiling point of the solvent.

18. Use of 10-bromosandwicin or 10-bromoisosandwicin for the preparation of $N_b$-quaternary 10-bromosandwicin or 10-bromoisosandwicin-derivatives.

19. 10-Bromosandwicin or 10-bromoisosandwicin and acid addition salts thereof with pharmacologically acceptable acids for use as medicaments.

**Revendications**

1. 10-bromosandwicine de formule I

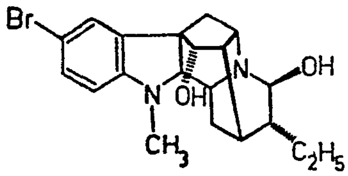

(I)

et ses sels d'addition avec des acides pharmacologiquement acceptables.

2. 10-bromoisosandwicine de formule II

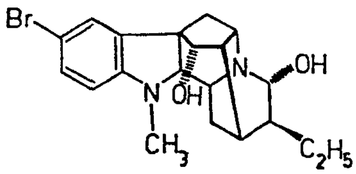

(II)

et ses sels d'addition avec des acides pharmacologiquement acceptables.

3. Procédé pour la préparation de 10-bromosandwicine, caractérisé en ce qu'on brome la sandwicine avec un agent de bromation approprié.

4. Procédé selon la revendication 3, caractérisé en ce qu'on brome dans un solvant inerte.

5. Procédé selon l'une des revendications 3 ou 4, caractérisé en ce qu'on brome avec du brome élémentaire.

6. Procédé selon l'une des revendications 3 ou 4, caractérisé en ce qu'on brome avec du bromure de cuivre (II) anhydre.

7. Procédé selon l'une des revendications 3 ou 4, caractérisé en ce qu'on brome avec de la 2,4,4,6-tétrabromo-2,5-cyclohexadiène-1-one.

8. Procédé selon l'une quelconque des revendications 3, 4 ou 5, caractérisé en ce qu'on brome dans un mélange de chlorure de méthylène et de méthanol.

9. Procédé selon l'une quelconque des revendications 3, 4 ou 6, caractérisé en ce qu'on brome dans le diméthylformamide.

10. Procédé selon l'une quelconque des revendications 3, 4 ou 7, caractérisé en ce qu'on brome dans un mélange de tétrahydrofuranne et de chlorure de méthylène auquel on peut ajouter du méthanol.

11. Procédé selon l'une des revendications 5 ou 8, caractérisé en ce qu'on brome à 0 °C.

12. Procédé selon l'une des revendications 6 ou 9, caractérisé en ce qu'on brome à la température ambiante.

13. Procédé selon l'une des revendications 7 ou 10, caractérisé en ce qu'on brome en refroidissant, de préférence entre − 5 et − 10 °C.

14. Procédé pour la préparation de 10-bromoisosandwicine, caractérisé en ce qu'on isomérise la 10-bromosandwicine dans des conditions alcalines.

15. Procédé selon la revendication 14, caractérisé en ce qu'on isomérise dans une lessive alcaline alcoolique.

16. Procédé selon la revendication 15, caractérisé en ce qu'on isomérise avec une lessive de potasse méthanolique.

17. Procédé selon la revendication 16, caractérisé en ce qu'on isomérise à la température d'ébullition du solvant.

18. Utilisation de la 10-bromosandwicine ou de la 10-bromoisosandwicine pour la préparation de dérivés $N_b$-quaternaires de 10-bromosandwicine ou de 10-bromoisosandwicine.

19. 10-bromosandwicine ou 10-bromoisosandwicine et leurs sels d'addition avec des acides pharmacologiquement acceptables, utilisables comme médicaments.